# EUROPÄISCHE PATENTSCHRIFT

(11) **EP 0 753 285 B1**
(45) Veröffentlichungstag und Bekanntmachung des Hinweises auf die Patenterteilung: **17.10.2001**
(21) Anmeldenummer: 96105977.1
(22) Anmeldetag: 17.04.1996
(51) Int. Cl.: A61B 6/08

(54) **Positionierung und Markierung eines Patienten an Diagnose- und Therapiegeräten**
Positioning and marking of a patient for diagnostic and therapeutic equipment
Positionnement et marquage d'un patient pour appareils diagnostiques et thérapeutiques

(30) Priorität: 08.07.1995 DE 19524951
(43) Veröffentlichungstag der Anmeldung: 15.01.1997
(73) Patentinhaber: LAP GmbH Laser Applikationen, D-21337 Lüneburg (DE)
(72) Erfinder: Hofmann, Karsten, 21354 Bleckede (DE)
(74) Vertreter: Patentanwälte Hauck, Graalfs, Wehnert, Döring, Siemons

(56) Entgegenhaltungen:
- EP-A- 0 077 899
- EP-A- 0 480 035
- EP-A- 0 687 443
- US-A- 4 242 587
- US-A- 4 629 989

## Beschreibung

Die Erfindung bezieht sich auf ein Verfahren zur Markierung eines zu bestrahlenden Gebietes.

In der Strahlentherapie ist es notwendig, den Strahl der Bestrahlungsquelle präzise auf das zu bestrahlende Gebiet eines Patientenkörpers auszurichten, und zwar genau reproduzierbar. Zur Verringerung der Belastung nicht zu therapierender Bereiche wird die Strahlungsquelle um ein sogenanntes Isozentrum geschwenkt, so daß im Zentrum des zu bestrahlenden Gebietes stets eine gleiche Dosisleistung erzielt wird, im benachbarten nicht zu behandelnden Gebiet hingegen eine deutlich verringerte Belastung stattfindet. Ein Patient ist daher im Hinblick auf das Bestrahlungsgerät so auszurichten, daß das Zentrum des zu bestrahlenden Gebietes mit dem Isozentrum des Bestrahlungsgerätes zusammenfällt. Die Lage des zu bestrahlenden Gebietes kann durch geeignete Diagnosemethoden, beispielsweise mit einem Computertomographen (CT) ermittelt werden. Dabei sind nicht nur die Koordinaten des Zentrums des zu bestrahlenden Gebietes wesentlich, sondern auch die Ausdehnung des Gebietes sowie der Umfang, der vom Bestrahlungsgerät erfaßt werden soll. Der Fokus des Bestrahlungsgerätes liegt üblicherweise zwischen Strahlungsquelle und dem Körper des Patienten. Dadurch ergibt sich eine divergente Strahlung, die je nach den Abmessungen des Körpers des Patienten eine mehr oder weniger große Fläche auf der Haut abdeckt. Mit Hilfe einer entsprechenden Maskierung kann erreicht werden, daß diese Fläche verkleinert wird.

Zur entsprechenden Ausrichtung des Patienten im Hinblick auf das Bestrahlungsgerät ist es notwendig, die Lage des zu bestrahlenden Gebietes anzuzeigen bzw. zu markieren. Es ist bekannt, mit Hilfe von in einem Bestrahlungsraum fest angeordneten Linienlasersystemen den Patienten auf einer Liege in eine präzise Lage zu bringen, bevor er in das Bestrahlungsgerät gefahren wird.

Die Koordinaten des zu bestrahlenden Gebietes werden mit Hilfe zum Beispiel eines Computertomographen ermittelt. Die Koordinaten werden auf der Haut des Patientenkörpers so markiert, daß bei einer Ausrichtung des Patienten zum Bestrahlungsgerät das Zentrum des Tumors im Isozentrum des Bestrahlungsgerätes liegt.

Das Bestrahlungsgebiet muß so gewählt werden, daß der in der Tiefe des Körpers befindliche Tumor in seiner vollen Ausdehnung von der Strahlung getroffen wird und das umliegende Gewebe weitestgehend geschont wird. Dazu ist es sinnvoll, eine Projektion des zu bestrahlenden Gebietes auf die Haut vorzunehmen, und zwar so, daß die Projektion der Laserlinien aus der bei der Therapieplanung gewählten Blickrichtung des Bestrahlungsgerätes erfolgt (beam's eye view), weil so die Hautoberfläche und der in der Tiefe des Körpers befindliche Tumor in einer Achse liegen und die Divergenz der Bestrahlungsquelle berücksichtigt werden kann und weil sonst die von den Lasern projizierten Linien durch die Körperkonturen verzerrt werden.

Aus EP-A-0 480 035 ist bekannt geworden, das Zentrum des zu bestrahlenden Areals zu markieren und ein perspektivisches Bild aus dem Blickwinkel der Strahlungsquelle zu errechnen. Diese dient zur optimalen Einstellung der Position und des Kollimators der Strahlungsquelle.

Der Erfindung liegt die Aufgabe zugrunde, ein Verfahren zur Markierung eines zu bestrahlenden Gebietes anzugeben.

Die Aufgabe wird durch die Merkmale nach Anspruch 1 gelöst.

Es ist üblich, daß die Bestrahlungsquelle in bezug auf die Körperachse des Patienten ortsfest ist, jedoch in einer Transversalebene rotiert werden kann. Dabei beschreibt der Fokus des Bestrahlungsgerätes einen Flugkreis um das Isozentrum. Der Patient kann auf einer beweglichen Liege in eine beliebige Position zum Bestrahlungsgerät und somit der Tumor in das Isozentrum gebracht werden. Um die Bestrahlungsquelle auf den Tumor auszurichten und um die Bestrahlung zu kontrollieren, ist es vorteilhaft, für eine gewünschte Einstellung der Bestrahlungsquelle bzw. einen gewünschten Verlauf der Bestrahlungsstrahlen die bestrahlte Hautfläche zu markieren. Vorzugsweise wird diese Markierung durch das folgende, weitere Verfahren vorgenommen, nachdem die Koordinaten des Tumors bezüglich seiner Lage und seiner Ausdehnung beispielsweise mit einem Computertomographen ermittelt worden sind.

Nach Ermittlung von Lage und Ausdehnung des zu bestrahlenden Gebietes des Patienten im Rahmen der Therapieplanung werden der Abstand zwischen dem Fokus des Bestrahlungsgerätes und dem Zentrum des zu bestrahlenden Bereiches, die Bestrahlungsrichtung und die Divergenz der Strahlung gewählt bzw. berechnet. Damit sind die Koordinaten des Fokus und der Verlauf der Bestrahlungsstrahlen festgelegt. Anschließend wird ein erster Linienlaserstrahl erzeugt, der durch den Fokus verläuft und in Verlängerung den zu bestrahlenden Bereich tangiert. Dieser Laserstrahl entspricht einem der Randstrahlen der Bestrahlungsstrahlen bei idealer Bestrahlung. Ferner wird ein zweiter Linienlaserstrahl erzeugt, der ebenfalls durch den Fokus verläuft und den zu bestrahlenden Bereich an etwa einer gegenüberliegenden Stelle tangiert. Dieser Linienlaserstrahl entspricht dem weiteren gewünschten Randstrahl der Bestrahlungsstrahlen. Zusammen mit den weiteren Strahlen der jeweiligen Linienlaserstrahlebene werden auf diese Weise zwei Markierungslinien auf der Haut des Patienten erzeugt. Deren Abstand in der durch die beiden Linienlaserstrahlen aufgespannten Ebene entspricht der Ausdehnung der zu bestrahlenden Hautfläche. Der Abstand der Markierungslinien und die Position der zu bestrahlenden Hautfläche entlang der Markierungslinien kann mit Hilfe eines Linienlasers angezeigt werden, der den Verlauf der durch die beiden Linienlaserstrahlen aufgespannten Ebene auf der Haut in Form einer weiteren Markierungslinie darstellt. Die Ausdehnung der zu bestrahlenden Hautfläche ist dann durch die Strecke zwischen den Punkten gegeben, in denen die ersten beiden Markierungslinien von der weiteren Markierungslinie geschnitten werden.

Vorzugsweise werden die Linienlaser bei dem Verfahren um eine zu der Sagittalachse parallele Achse geschwenkt. Somit können die Strahlenebenen so ausgerichtet werden, daß die Strahlen in den Transversalebenen nicht parallel oder orthogonal zu den Achsen des Koordinatensystems verlaufen, sondern einen beliebigen Winkel mit diesen bzw. den Verfahrachsen einnehmen können.

Ferner können die Linienlaser um eine in der Linienstrahlebene parallel zu den Linienstrahlen verlaufende Achse gedreht werden. Auf diese Weise kann erreicht werden, daß beispielsweise durch eine Drehung um 90° ein Linienlaser anstelle einer zu der Sagittalachse parallelen Linie eine zu der Transversalachse parallele Linie auf der Haut des Patienten erzeugt.

Ein wichtiger Vorteil dieses Verfahrens besteht darin, daß die Linienlaserstrahlen wie Randstrahlen der Bestrahlungsstrahlen verlaufen, also auch durch den Fokus des Bestrahlungsgerätes gehen, und somit die Divergenz der Bestrahlungsstrahlen und der Linienlaserstrahlen gleich ist. Damit ist diese Simulation der Bestrahlungsstrahlen durch die Linienlaserstrahlen unabhängig von dem Abstand des Fokus und der Hautoberfläche des Patienten. Es entsteht also kein Parallaxenfehler.

Die Freiheitsgrade der Linienlaser sind insbesondere bei Verwendung eines Computertomographen zur Diagnose von Vorteil, da bei einem Computertomographen die Patientenliege nur in Richtung zweier Achsen bewegt werden kann, nämlich vorwärts und rückwärts und nach oben und nach unten.

Ein weiterer Vorteil besteht darin, daß der mechanische und steuerungstechnische Aufwand das Verfahren geringer sind, als wenn die Linienlaser frei im Raum beweglich wären, um den Strahlungsverlauf der Bestrahlungsquelle zu simulieren. Vorzugsweise wird die Positionierung und die Winkeleinstellung der Linienlaser durch einen mit einem Steuerungs-Rechner durchgeführten Algorithmus vorgenommen, der den Verlauf der Linienlaserstrahlen aus den durch die Diagnose und die Therapieplanung vorgegebenen Daten errechnet. Diese Daten können vom Therapieplanungssystem an den Steuerungs-Rechner für die Positionier-Steuervorrichtung oder über das Eingabegerät an diese gegeben werden. Die Positionier-Steuervorrichtung steuert die numerischen Antriebe für die Linienlaser, um die gewünschten Bewegungen herbeizuführen.

Die Erfindung wird nachfolgend anhand von Zeichnungen näher erläutert.
- Fig. 1: zeigt schematisch eine Anordnung schwenkbarer Linienlaser zusammen mit einem Computertomographen zur Durchführung des Verfahrens zur Markierung eines zu bestrahlenden Gebietes.
- Fig. 2: verdeutlicht schematisch das Prinzip des Verfahrens zur Markierung eines zu bestrahlenden Gebietes.

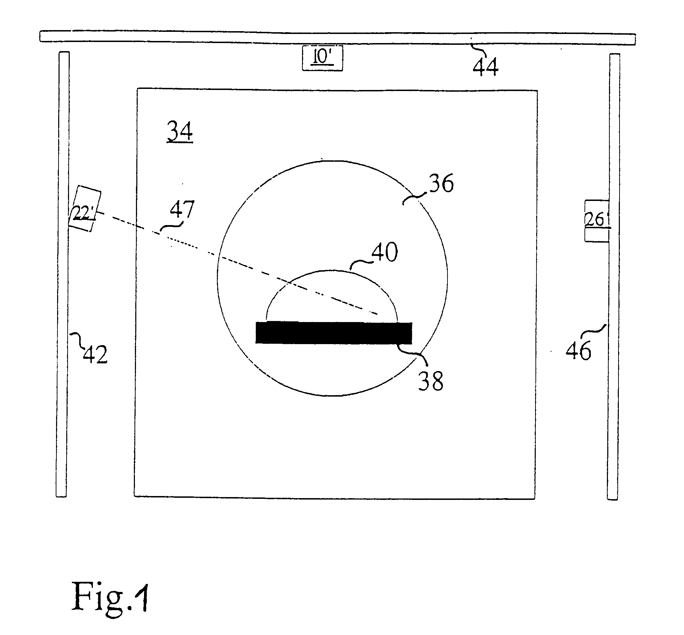

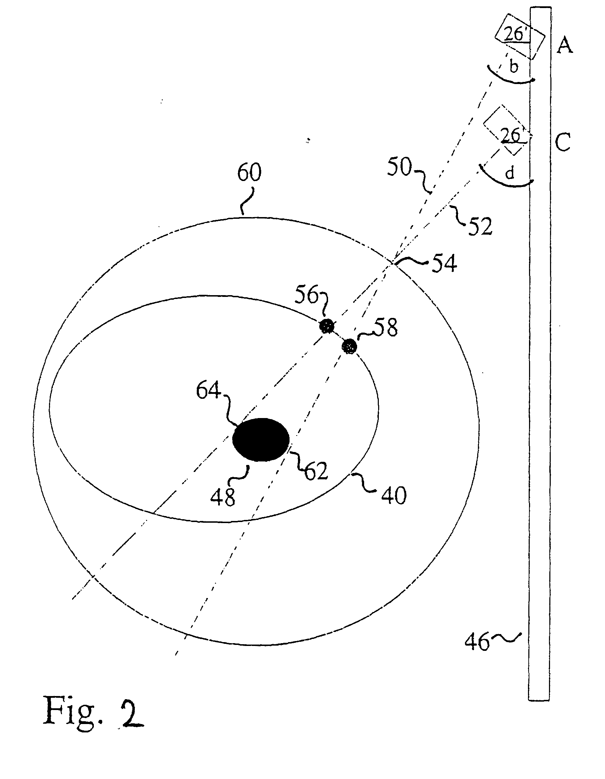

Eine Anordnung von Linienlasern, bei der diese zusätzlich zur Verfahrbarkeit auch schwenkbar sind, ist in den Fig. 1 und 2 gezeigt.

In Fig. 1 ist ein Computertomograph 34 mit einer Öffnung 36 dargestellt, in der sich eine Patientenliege 38 mit einem Patienten 40 befindet. Die Liege ist in vertikaler Richtung und in Längsrichtung verfahrbar. Linienlaser 10', 22' und 26' sind jeweils an einer von drei linearen Verfahrschienen 42, 44 und 46 angebracht, die in einer rechtwinkligen Anordnung portalartig um den Computertomographen herum angeordnet sind. Die Linienlaser 10', 22', 26' sind um eine zu der durch die Verfahrschienen 42, 44, 46 aufgespannten Ebene senkrechte Achse schwenkbar, wie dies für den Linienlaser 22' und einen in der Transversalebene verlaufenden Strahl 47 angedeutet ist. Die dargestellten Linienlaser können Strahlebenen erzeugt werden, die sich unter einem von 90° verschiedenen Winkel schneiden.

Fig. 4 zeigt die Verfahrschiene 46 mit dem Linienlaser 26', der in zwei unterschiedlichen Positionen A und C an der Verfahrschiene 46 dargestellt ist. Der Linienlaser 26' nimmt in der Position A mit der Verfahrschiene 46 einen Schwenkwinkel b ein und in der Position C einen Schwenkwinkel d ein. Die Laserstrahlen sind auf einen Patienten 40 mit einem Tumor 48 ausgerichtet. Zwei Strahlen 50 und 52, die Teil der jeweils von dem Laser in den beiden Positionen A und C erzeugten Strahlebenen sind und innerhalb einer durch den Tumor 48 gehenden Transversalebene verlaufen, schneiden sich in einem Punkt 54 und treffen in den Punkten 56 und 58 auf den Patienten 40. Der Schnittpunkt 54 liegt auf einem Kreis 60, auf dem der Fokus eines Bestrahlungsgerätes (nicht gezeigt) bewegt werden kann. Der Mittelpunkt dieses Flugkreises 60 fällt mit dem Zentrum des Tumors 48 zusammen. Der Linienlaserstrahl 50 ist so ausgerichtet, daß er in seiner Verlängerung den Tumor 48 an einer Stelle 62 tangiert, und der Linienlaserstrahl 52 ist so ausgerichtet, daß er in seiner Verlängerung den Tumor an einer weiteren Stelle 64 tangiert.

Nachfolgend wird das Verfahren zur Markierung eines zu bestrahlenden Gebietes unter Anwendung der Vorrichtung nach Fig. 1 und 2 beschrieben. Nachdem die Lage und die Ausdehnung des Tumors 48 des Patienten 40 mit dem Computertomographen 34 ermittelt worden sind, werden die Patientenliege und die Position der Laser so verändert, daß das Zentrum des Tumors 48 die Koordinaten des Mittelpunktes des Fokusflugkreises 60 des später zu positionierenden Bestrahlungsgerätes einnimmt. Damit gelangt der Tumor in das Isozentrum des Bestrahlungsgerätes. Der Linienlaser 26' wird nun verwendet um die Hautfläche des Patienten 40 zu markieren, die gemäß einer vorgenommenen Therapieplanung von den Bestrahlungsstrahlen getroffen werden soll. Dazu wird der Linienlaser 26' so verfahren und geschwenkt, daß er einen Strahl 50 innerhalb der durch den Fokusflugkreis bestimmten Transversalebene aussendet, der mit einem gewünschten Randstrahl der Bestrahlungsstrahlen zusammenfällt, was gleichbedeutend damit ist, daß der Strahl 50 durch den Fokus 54 verläuft und in Verlängerung den Tumor 48 an der Stelle 62 tangiert. Anschließend wird der Linienlaser 26' so verfahren und geschwenkt, daß er einen weiteren Strahl 52 erzeugt, der den anderen gewünschten Randstrahl der Bestrahlungsstrahlen innerhalb der Transversalebene darstellt, was dadurch erreicht wird, daß der Strahl 52 ebenfalls durch den Fokus 54 verläuft und in Verlängerung den Tumor 48 an einer anderen Stelle 64 tangiert. Die Linienlaserstrahlen 50, 52 treffen somit in den Punkten 56 und 58 auf die Haut des Patienten, in denen auch später die Randstrahlen des Bestrahlungsstrahlen innerhalb der gewählten Transversalebene auf die Haut treffen werden. Die Punkte 56 und 58 liegen innerhalb von Projektionslinien, die von den Strahlebenen des Linienlasers 26' auf die Haut projiziert werden. Die Punkte 56 und 58 ergeben sich als Schnittpunkte dieser Markierungslinien mit der Transversalebene. Zur Kennzeichnung der Punkte 56, 58 kann die Transversalebene durch einen weiteren Linienlaser (nicht gezeigt), der eine Transversallinie erzeugt, dargestellt werden. Die so auf der Haut abgebildeten Schnittpunkte 56, 58 werden zur Markierung des Patienten genutzt.

## Patentansprüche

1. Verfahren zur Markierung eines mit einer Bestrahlungsquelle zu bestrahlenden Gebietes (48), dessen Lage und Ausdehnung beispielsweise mittels eines Computertomographen ermittelt werden, wobei für die Bestrahlungsquelle die Bestrahlungsrichtung, der Abstand zwischen Fokus (54) und einem Zentrum des zu bestrahlenden Gebietes (48) sowie die Divergenz und deren Richtung gewählt sind, **gekennzeichnet durch** folgende Verfahrensschritte :
- ein erster **durch** den Fokus (54) laufender Linienlaserstrahl (50) wird erzeugt, der in einer Verlängerung das zu bestrahlende Gebiet tangiert,
- ein zweiter **durch** den Fokus (54) laufender Linienlaserstrahl (52) wird erzeugt, der ebenfalls **durch** den Fokus (54) geht und dessen Verlängerung das zu bestrahlende Gebiet an einer anderen Stelle tangiert,
- wodurch zwei Markierungslinien auf der Haut des Patienten erzeugt werden, deren Abstand in der **durch** die beiden Strahlen (50, 52) aufgespannten Ebene, der Ausdehnung der zu bestrahlenden Hautfläche entspricht.

2. Verfahren nach Anspruch 1, **dadurch gekennzeichnet, daß** der erste und zweite Linienlaserstrahl von einem Linienlaser (10', 22', 26') nacheinander erzeugt werden, wobei der Linienlaser dazu entlang einer Verfahrschiene verfahren und geschwenkt wird.

3. Verfahren nach Anspruch 1 oder 2, **dadurch gekennzeichnet, daß** die durch die beiden Linienlaserstrahlen (50, 52) aufgespannte Ebene auf der Haut des Patienten durch eine mittels eines Linienlaser erzeugte weitere Markierungslinie dargestellt wird, die mit den anderen beiden Markierungslinien Schnittpunkte bildet.

4. Verfahren nach einem der Ansprüche 1 bis 3, **dadurch gekennzeichnet, daß** die Linienlaser (10', 22', 26') um eine zu der Sagittalachse parallele Achse geschwenkt werden.

5. Verfahren nach einem der Ansprüche 1 bis 4, **dadurch gekennzeichnet, daß** die Linienlaser um eine in der Linienstrahlebene parallel zu den Linienstrahlen verlaufende Achse gedreht werden.

## Claims

1. A method of marking a region (48) to be irradiated by an radiation source the position and extension of which, for instance, are determined by means of a computer tomograph wherein the direction of radiation, the distance between the focus (54) and a centre of the region (48) to be irradiated and the divergence and the direction thereof are chosen for the radiation source, **characterized by** the steps of:
- generating a first line laser beam (50) which passes through the focus (54) and is tangent to the region to be irradiated in an extension,
- generating a second line laser beam (54) which also passes through the focus (54) and the extension of which is tangent to the region to be irradiated in another point,
- which produces two marking lines on the patient's skin the distance between which lines in the plane spanned by the two beams (50, 52) corresponds to the extension of the skin region to be irradiated.

2. The method according to claim 1, **characterized in that** the first and second line laser beams are successively produced by a line laser (10', 22', 26') with the line laser being moved along a displacement rail and being pivoted for this purpose.

3. The method according to claim 1 or 2, **characterized in that** the plane spanned by the two line laser beams (50, 52) on the patient's skin is shown by another marking line produced by means of a line laser, which forms intersections with the two other marking lines.

4. The method according to any one of claims 1 to 3, **characterized in that** the line lasers (10', 22', 26') are pivoted about an axis in parallel with the sagittal axis.

5. The method according to any one of claims 1 to 4, **characterized in that** the line lasers are rotated around an axis extending in the line beam plane in parallel with the line beams.

## Revendications

1. Procédé de marquage d'une zone (48) devant être irradiée avec une source de rayonnement et dont la position et l'étendue sont déterminées, par exemple, au moyen d'un tomographe commandé par ordinateur, la direction du rayonnement, la distance entre le foyer (54) et un centre de la zone à irradier (48), ainsi que la divergence et sa direction, étant choisis pour la source de rayonnement,
**caractérisé par** les pas de procédé suivants:
- un premier rayon laser linéaire (50) est produit, passant par le foyer (54), et qui, dans sa prolongation, est tangent à la zone à irradier,
- un deuxième rayon laser linéaire (52), passant par le foyer (54), et qui, de même, passe par le foyer (54) et dont la prolongation est tangente à la zone à irradier, en un autre point,
- deux lignes de marquage étant ainsi créées sur la peau du patient, dont la distance, dans le plan déterminé par les deux rayons (50, 52), correspond à l'étendue de la surface de peau à irradier.

2. Procédé suivant la revendication 1, **caractérisé en ce que** les premier et deuxième rayons laser linéaires sont produits l'un après l'autre par un laser linéaire (10', 22', 26'), en déplaçant, pour cela, le laser linéaire le long d'un rail de déplacement et en le faisant pivoter.

3. Procédé suivant la revendication 1 ou la revendication 2, **caractérisé en ce que** le plan défini par les deux rayons laser linéaires (50, 52) est représenté, sur la peau du patient, par une autre ligne de marquage, produite au moyen d'un laser linéaire, cette ligne de marquage formant avec les deux autres lignes de marquage, des points d'intersection.

4. Procédé suivant l'une des revendications 1 à 3, **caractérisé en ce que** l'on fait pivoter les lasers linéaires (10', 22', 26') autour d'un axe parallèle à l'axe sagittal.

5. Procédé suivant l'une des revendications 1 à 4, **caractérisé en ce qu'**on fait tourner les lasers linéaires autour d'un axe s'étendant dans le plan des rayons laser et dirigé parallèlement aux rayons laser.
